# EUROPEAN PATENT SPECIFICATION

(11) **EP 2 934 654 B1**
(45) Date of publication and mention of the grant of the patent: **21.06.2017**
(21) Application number: 13824395.1
(22) Date of filing: 16.12.2013
(51) Int. Cl.: A61M 25/06, A61M 5/50

(54) **CANNULA-NEEDLE WITH PROTECTIVE MEMBER**
KANÜLENNADEL MIT SCHUTZELEMENT
CANULE-AIGUILLE AVEC ÉLÉMENT DE PROTECTION

(30) Priority: 20.12.2012 IT MO20120309
(43) Date of publication of application: 28.10.2015
(73) Proprietor: Delta Med S.p.A., 46019 Viadana (IT)
(72) Inventor: BERTOLI, Alessandro, I-26037 San Giovanni In Croce (CR) (IT); BALBONI, Alessandro, I-46030 San Giorgio Di Mantova (MN) (IT)
(74) Representative: Bergamini, Silvio
(86) International application number: PCT/IB2013/060978
(87) International publication number: WO 2014/097110

(56) References cited:
- WO-A1-2004/000408
- WO-A1-2004/093961
- US-A1- 2005 075 609

## Description

### Field of the invention

The invention relates to a cannula-needle with a protective member, which is generally used to allow health care operators to handle a cannula-needle with the utmost safety without any risk of being accidentally pricked.

### Background art

A number of medical apparatus are known which comprise a needle for making injections into blood vessels of patients, and which are equipped with protective devices to prevent operators from being accidentally injured after use.

Particularly, these safety devices are used to prevent transmission of infectious diseases through accidental punctures from a patient to health care operators who treat him/her and provide the required therapy.

Typically, prior art protective devices consist in specially shaped springs or ratchet gears, which are designed to be disabled when the needle is used, and are enabled after needle use, i.e. once the needle has been extracted from the blood vessel of the patient after the injection.

More in detail, prior art protective devices are arranged to slide on a needle stem and irreversibly come to a position in which they entirely cover the needle tip.

These devices are placed in special housing elements, typically in the form of box-like shells, which contain them and allow the operators to grab and slide them along the needle stem from the disabled position to the active protection position.

Further prior art protective devices are designed for automatic actuation during needle withdrawal from the blood vessel of the patient.

Also in this case, the safety device is held within a box-like shell which is arranged, like in the previous cases, between a proximal end of the needle and the needle-holder, and hence is an additional element that is always present on the apparatus, both during and after use thereof.

From WO2004/093961 a safety needle and catheter assembly is known.

The catheter assembly has an introducer needle with proximal end and distal ends attached to a needle hub and a bent area therebetween.

The device further includes a tubular catheter having proximal and distal ends in which the introducer needle can be coaxially received within the catheter.

The device has a hollow catheter hub having a distal end attached to the proximal end of the catheter and is in fluid communication therewith.

The assembly includes a needle tip protector having a proximal end and a distal end disposed within the catheter hub.

The distal end of the protectors covers the needle tip when the needle is removed from the catheter.

The protector has a proximal opening at his proximal end wherein the bent area is angled away from the proximal opening such that when the needle is removed from the catheter the protector remains attached to the needle.

From WO2004/000408 a catheter and introducer needle assembly with needle shield is known.

The assembly has a distal tip and a static feature. The needle shield assembly includes an adapter having an open distal terminus and an open proximal terminus to allow the passage of the needle and a needle shield slidably associated with the adapter having an open distal end and an open proximal end where the latter is sufficiently narrow to restrict proximal movements of the needle static feature causing the shield to move in a proximal direction when the needle is pulled proximally after the static feature has established contact with the needle shield proximal end.

The assembly includes a canting plate having an unactivated first position and an activated second position that restricts needle movement.

The canting plate is activated via a canting plate retention system in communication with the canting plate and responsive to proximal movement of the needle.

The above described prior art suffers from certain drawbacks.

A first drawback consists in that these protective devices always require a box-like shell that contains them.

This will considerably increase the manufacturing costs for apparatus having protective devices, both because they require a special shell to contain the safety device and because safety devices must be assembled and inserted into their respective shells.

A second drawback is that, even when shells have small sizes, they are still provided on the needle stem as additional bodies, thereby limiting handling freedom by health care operators.

It should be noted that the needles that are typically used in the medical field to make injections into the blood vessels of patients are very small and that the operators are required to wear protective gloves that limit finger sensitivity and hence, grip safety.

Therefore, the presence of an additional element is an additional restriction to freedom of movement, as mentioned above.

### Disclosure of the invention

The invention has the object to improve the prior art.

In one aspect, the invention relates to a cannula-needle with a protective member according to the features of claim 1.

The invention achieves the following advantages:
- providing a cannula-needle that contains the protective member therein and avoids the need for additional container bodies;
- improving handling of the cannula-needle, as no container for the protective member is interposed between the needle-holder and the needle;
- affording automatic actuation of the protective member during withdrawal of the needle from the cannula, once the latter has been inserted into a blood vessel of a patient.

### Brief description of the drawings

Further features and advantages of the invention will be more readily apparent upon reading of the detailed description of preferred non-exclusive embodiments of a cannula-needle with a protective member, which is shown as a non-limiting example in the annexed drawings, in which:
FIG. 1 is a highly schematic and magnified broken view of a longitudinal section of a cannula-needle of the invention, in a configuration of use, as taken along a plane I-I of Figure 14;
FIG. 2 is a view of the cannula-needle of Figure 1, in a later configuration of use;
FIG. 3 is a detail view of a section of a needle stem upon which a tip protection member is mounted, in a disabled and cannula holder-free configuration, for clarity;
FIG. 4 is a view of the needle stem section of Figure 3, in an enabled configuration of the protective member;

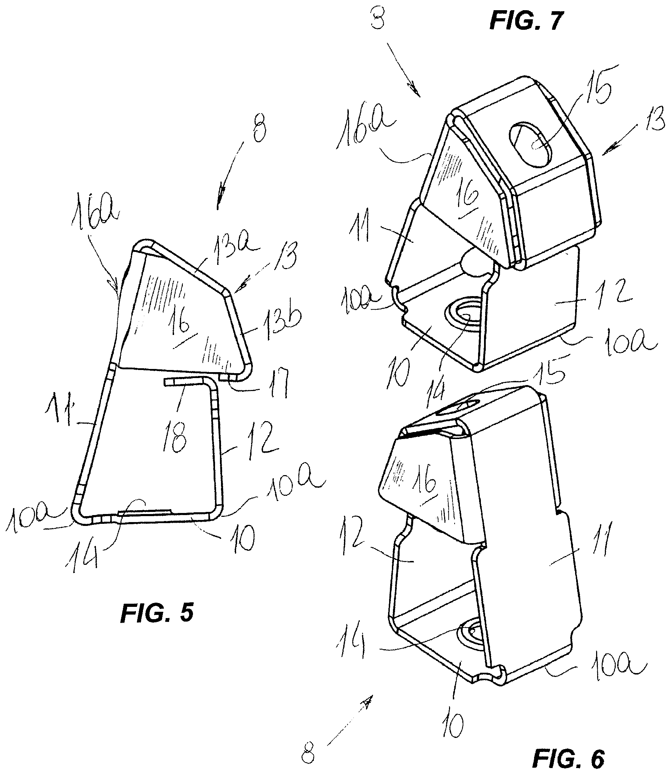

FIG. 5 is a side view of the protective member of the invention;
FIG. 6 is a perspective view of the protective member from a first point of view;
FIG. 7 is a perspective view of the protective member from a second point of view, opposite to that of Figure 6;
FIGS. 8a to 8d are schematic, smaller-scale views of the steps from a disabled state to an enabled state of the protective member of Figure 1;
FIG. 9 is a perspective view of a needle holder in which the protective member is in the enabled configuration;
FIG. 10 is a longitudinal sectional view of a cannula with a protective member mounted therein according to the invention, as taken along a plane X-X of Figure 14;
FIG. 11 is a longitudinal sectional magnified view of a detail of the cannula of Figure 10, as taken along a plane XI-XI of Figure 13.
FIG. 12 is a further magnified view of the protective member in an enabled configuration;
Figure 13 is a cross-sectional view of the cannula of Figure 10, as taken along a plane XIII-XIII of Figure 10;
FIG. 14 is a general, slightly smaller view of the cannula-needle of Figure 1, in an assembled, ready-for-use configuration.

### Detailed description of a preferred embodiment

Referring to the figures, numeral 1 generally designates a cannula-needle, which is typically designed for use to make injections into a blood vessel of a patient.

The cannula needle 1 comprises a needle holder 2 which supports a needle 3 having a longitudinal axis "A" and is designed to be coaxially inserted into a flexible cannula 4, to temporarily impart flexural strength thereto, and act as an introducer for the blood vessel to be introduced into the blood vessel without bending.

The cannula 4 is supported at the distal end by a cannula holder 5 and extends therefrom outwards.

The needle 3 forms a tip 3a at the distal end of the stem 3b and, proximate thereto, a transverse engagement member, namely a raised boss 9.

The cannula holder 5 has an axial inner cavity 6 formed therein, which is delimited by walls 7 and in which a protective member 8 is designed to be precisely received and removably retained.

This protective member 8 consists of a sheet-like body, which is typically made with a metal material, such as harmonic steel, and is shaped to form a proximal wall 10 transverse to the stem 3b of the needle 3 and two arms 11 and 12 extending from two opposite and folded edges 10a of the wall 10, and face toward the tip 3a.

The arm 11 is formed in such a manner that it may maintain a spontaneously oblique position relative to the arm 12, to tend and converge toward the latter and, as a result, toward the stem 3b of the needle 3.

Furthermore, the arm 11 forms a second wall 13, at the end opposite to the one that is connected to the wall 10, which is folded toward the arm 12 and is divided into two successive sections 13a and 13b, slightly bent relative to each other.

A first opening 14 is formed in the wall 10, which has a circular perimeter and is designed for the stem 3b of the needle 3 to slide therethrough.

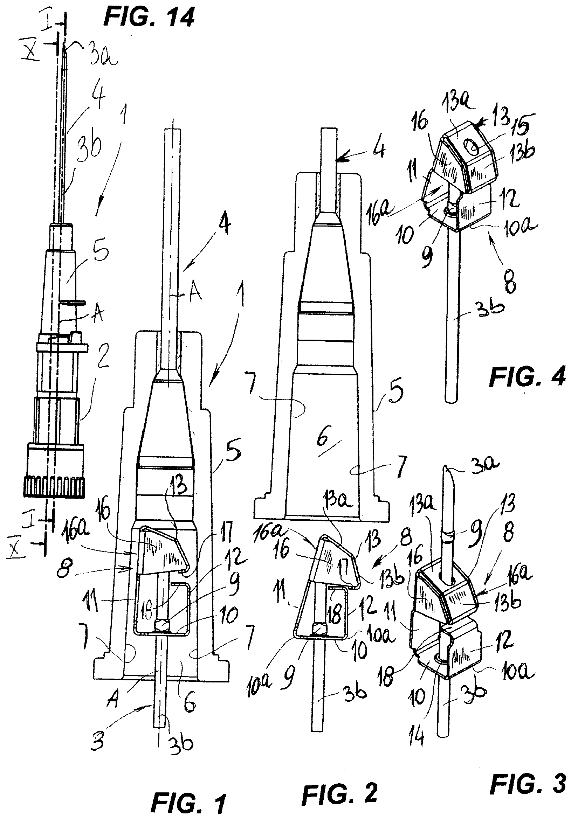

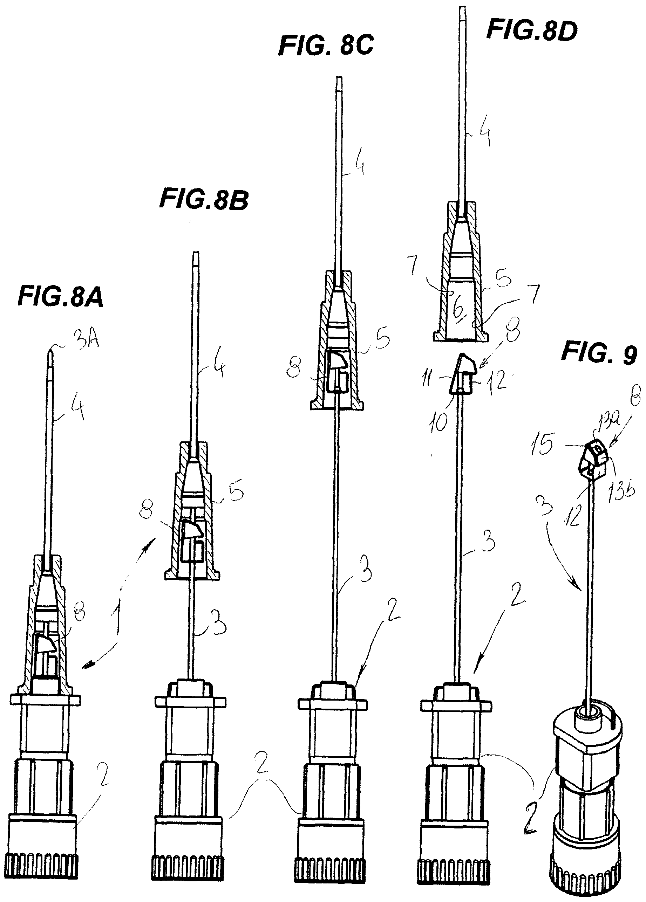

A second opening 15 is formed in the section 13a of the second wall, which is also designed for the stem 3b to extend therethrough, when the protective member 8 is in a disabled configuration, as shown in Figures 3, 8a, 8b, 8c.

The arm 11 also forms two lateral partitions 16 which are folded perpendicular thereto and form with the second wall 13 adjacent thereto, a sort of box-like head 16a, which nevertheless has an entirely open side facing the wall 10.

The second section 13b is formed with a lip 18 at its free end, which is folded toward the arm 11 and whose purpose is described below.

The arm 12 is substantially straight and stationary with respect to the proximal wall 10 and is substantially perpendicular thereto.

The arm 12 has a free distal end that forms a second lip 18 folded toward the arm 11 and hence toward the stem 3b of the needle 3.

It shall be noted that the diameter of the opening 14 is slightly smaller than the diameter of the boss 9 and for this reason, the boss 9 cannot pass through it when the needle 3 slides relative to the protective member 8.

Conversely, the second opening 15 has a slightly larger size than the outside diameter of the boss 9, such that the latter can pass through it when the needle 3 slides relative to the protective member 8.

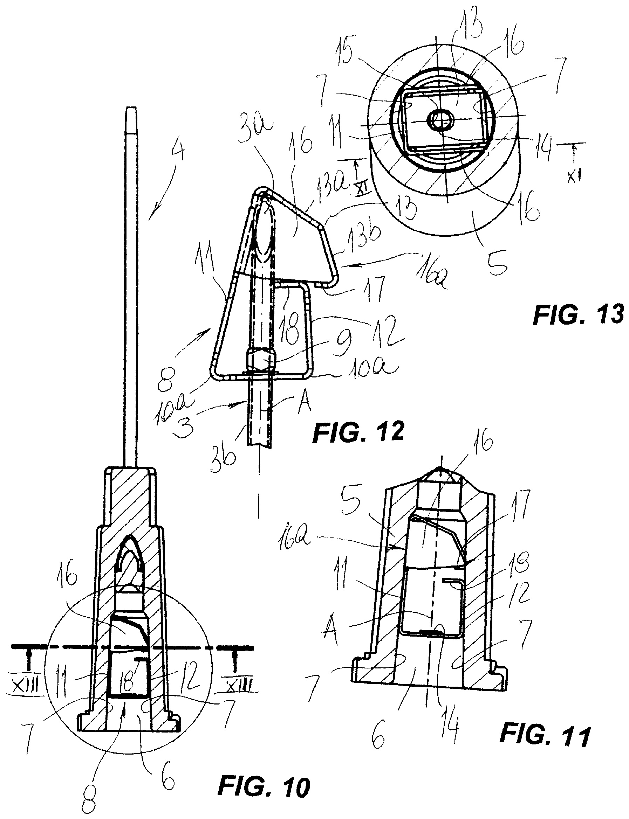

The protective member 8 is normally retained in the disabled configuration within the cavity 6, typically by adhesion between the edges of the lateral partitions 16 and the walls 7, as shown in greater detail in Figure 13.

When the cannula-needle 1 is not used, the stem 3b extends through both openings 14 and 15 and transversely pushes the arm 11, thereby forcing it to bend toward the wall 7 to a biased position substantially parallel to the arm 12.

This state is shown in schematic detail in Figures 1, 3 and more generally in Figures 8a, 8b, 8c.

After use of the cannula-needle 1, when the protective member 8 is in the enabled position, the arm 11 takes its natural oblique position relative to the proximal position 10 and the second wall 13 comes before the tip 10a, as shown in Figures 2 and 12.

The operation is as follows: when the cannula-needle 1 is in a non-use or preparation-for-use configuration, it is in the state as shown in Figure 8a.

More in detail, the protective member 8 is inserted in the axial cavity 6 and is retained therein by adhesion between the partitions 16 and the inner walls 7.

The needle 3 extends through both walls 10 and 13 of the protective member 8, after passing through the openings 14 and 15.

It shall be noted that the second opening 15 is slightly elliptical for alignment with the first opening 14 in this disabled state of the protective member 8, although the second wall 13 thereof is oblique to the proximal wall 10: this aligned state, which allows the needle 3 to slide, is shown in greater detail in Figure 13.

Once the health care operator has made an injection with the cannula-needle 1, by introducing it into a blood vessel of a patient, he/she withdraws the needle 3 from the cannula 4 by a withdrawal operation, i.e. by maintaining the cannula 4 in the blood vessel of the patient and simultaneously pulling out the needle in the proximal direction 3, while holding the needle holder 2.

In this pull-out operation, the stem 3b of the needle 3 progressively slides within the two openings 14 and 15 (see Figure 8b) until the boss 9 stops against the peripheral edge of the first opening 14, after passing through the second opening 15: this condition is shown in Figure 8c.

As the pull-out operation continues, the operator overcomes the adhesion force that retains the protective member 8 in the cavity 6 and progressively pulls it out with the needle 3, until it is entirely withdrawn from the cavity 6: this condition is shown in Figure 8d.

As the tip 3a of the needle 3 is pulled out of the protective member 8, moves beyond the second opening 15 and disengages therefrom, the arm 11 is instantaneously restored into its oblique position and the second opening 15 is finally and irreversibly out of alignment with the first opening 14 and with the tip 3a, which is entirely and automatically covered by the first section 13a of the second wall 13 (see Figure 12), whereby the operator is safeguarded against accidental pricks as he/she completes withdrawal and later disposes of the needle 3.

As shown in Figure 12, in the enabled configuration the protective member 8 is slightly inclined to the axis "A" of the stem 3b of the needle 3 and the second lip 18 abuts the stem 3b, thereby adding stability to the position of the protective member 8 on the needle 3.

The invention has been found to fulfill the intended objects.

The invention so conceived is susceptible to changes and variants within the inventive concept.

Also, all the details may be replaced by other technical equivalent elements.

In its practical implementation, any material, shape and size may be used as needed, without departure from the scope as defined by the following claims.

## Claims

1. A cannula-needle (1) comprising:
- A needle holder (2) that holds a needle (3) which is designed to be coaxially coupled with a cannula (4) for making an injection into a blood vessel and having a stem (3b) having a longitudinal axis (A), a distal tip (3a) and transversal engaging means (9) fitted in the proximity of said tip (3a);
- A cannula holder (5) in which said cannula (4) extends from its distal end and that at the opposing proximal end shapes an inner cavity (6) for coupling with said needle holder (2) and having inner walls (7);
- A protective member (8) of said needle tip (3a) which can be moved from a deactivated position to an irreversible activated position after the use of the cannula-needle (1) wherein it is placed over the needle tip (3a);
said protective member (8) in said deactivated position being retained in a removable way by said walls (7) of said inner cavity (6) of said cannula holder (5), said protective member (8) comprising a flat body which shapes:
- A crosswise proximal wall (10) to said needle wherein a first passage opening (14) for said stem (3b) is obtained and having a blocking perimeter of said transversal engaging means (9);
- A first and a second arm (11, 12) which extend from said proximal wall (10) toward said distal tip (3a);
At least said first arm (11) shaping a second wall (13) transversally fitted with respect of said needle (3) and wherein a second passage opening (15) of said stem (3b) is obtained, said second passage opening (15) in said deactivated position being aligned with said first passage opening (14) and in said activated position being misaligned from said first passage opening (14), **characterized in that** said first arm (11) forms two lateral partitions (16) which are folded perpendicular thereto and form with the second wall (13) adjacent thereto, a box-like head (16a) having an entirely open side facing the proximal wall (10)

2. A cannula-needle as claimed in claim 1, wherein between said inner cavity (6) and said protective member (8) lateral partitions (16) of said protective member (8) are interposed, so configured to restraint it in said deactivated position and to release it toward said activated position.

3. A cannula-needle as claimed in claim 2, wherein said retention means comprise a couple of lateral partitions (16) that extends from at least one of said first and second arms (11, 12) and converging toward said second wall (13), said lateral partitions (16) having respective gripping edges on said inner walls (7) of said inner cavity (6).

4. A cannula-needle as claimed in claim 1, wherein said first arm (11) in said activated position is normally oblique with respect of said transversal proximal wall (10).

5. A cannula-needle as claimed in claim 1, wherein said second arm (12) is normally perpendicular to said transversal proximal wall (10).

6. A cannula-needle as claimed in anyone of preceding claims, wherein said protective element (8) is slidingly withdrawn from said inner cavity (6) during the retraction of said needle (3), in contrast with said removable retention means (16), when said transversal engaging means (9) are blocked against said blocking perimeter of said first passage opening (14).

## Patentansprüche

1. Kanülennadel (1), umfassend:
- einen Nadelhalter (2), der eine Nadel (3) hält, welche ausgelegt ist, um mit einer Kanüle (4) zum Ausführen einer Injektion in ein Blutgefäß koaxial gekoppelt zu sein und mit einem Schaft (3b) mit einer Längsachse (A), einer distalen Spitze (3a) und transversalem Eingriffsmittel (9), eingepasst in Nachbarschaft zu der Spitze (3a);
- einen Kanülenhalter (5), in welchem sich die Kanüle (4) von ihrem distalen Ende erstreckt und der an dem gegenüberliegenden proximalen Ende einen inneren Hohlraum (6) zum Koppeln mit dem Nadelhalter (2) formt und innere Wände (7) aufweist;
- ein Schutzelement (8) der Nadelspitze (3a), das von einer deaktivierten Position zu einer irreversibel aktivierten Position nach der Anwendung der Kanülennadel (1) bewegt werden kann, wobei es über der Nadelspitze (3a) angeordnet ist;
wobei das Schutzelement (8) in der deaktivierten Position in einer entfernbaren Weise durch die Wände (7) des inneren Hohlraums (6) des Kanülenhalters (5) gehalten wird, wobei das Schutzelement (8) einen ebenen Körper umfasst, welcher formt:
- eine zu der Nadel querlaufende proximale Wand (10), wobei eine erste Durchgangsöffnung (14) für den Schaft (3b) erhalten wird und einen blockierenden Umfang des transversalen Eingriffsmittels (9) aufweist;
- einen ersten und einen zweiten Arm (11, 12), die sich von der proximalen Wand (10) zu der distalen Spitze (3a) erstrecken;
wobei mindestens der erste Arm (11) eine zweite Wand (13) transversal eingepasst hinsichtlich der Nadel (3) ausbildet und wobei eine zweite Durchgangsöffnung (15) des Schafts (3b) erhalten wird, wobei die zweite Durchgangsöffnung (15) in der deaktivierten Position mit der ersten Durchgangsöffnung (14) ausgerichtet ist und in der aktivierten Position von der ersten Durchgangsöffnung (14) verschoben ist, **dadurch gekennzeichnet, dass** der erste Arm (11) zwei seitliche Teilungen (16) bildet, die rechtwinklig dazu gefaltet sind und mit der zweiten Wand (13) benachbart dazu einen kastenartigen Kopf (16a) mit einer vollständig offenen Seite, die zu der proximalen Wand (10) weist, bilden.

2. Kanülennadel nach Anspruch 1, wobei zwischen dem inneren Hohlraum (6) und dem Schutzelement (8) Retentions-seitliche Teilungen (16) des Schutzelements (8) dazwischen angeordnet sind, so ausgelegt, um es in der deaktivierten Position zurückzuhalten und es zu der aktivierten Position zu lösen.

3. Kanülennadel nach Anspruch 2, wobei das Haltemittel ein Paar von seitlichen Teilungen (16) umfasst, das sich von mindestens einem von dem ersten und zweiten Arm (11, 12) erstreckt und sich der zweiten Wand (13) annähert, wobei die seitlichen Teilungen (16) jeweilige greifende Kanten an den inneren Wänden (7) des inneren Hohlraums (6) aufweisen.

4. Kanülennadel nach Anspruch 1, wobei der erste Arm (11) in der aktivierten Position hinsichtlich der transversalen proximalen Wand (10) normalerweise geneigt ist.

5. Kanülennadel nach Anspruch 1, wobei der zweite Arm (12) zu der transversalen proximalen Wand (10) normalerweise rechtwinklig ist.

6. Kanülennadel nach einem der vorstehenden Ansprüche, wobei das Schutzelement (8) von dem inneren Hohlraum (6) während des Zurückziehens der Nadel (3) gleitend weggezogen wird, im Gegensatz zu dem entfernbaren Retentionsmittel (16), wenn die transversalen Eingriffsmittel (9) gegen den blockierenden Umfang von der ersten Durchgangsöffnung (14) blockiert sind.

## Revendications

1. Canule-aiguille (1) comprenant :
- un porte-aiguille (2), portant une aiguille (3) conçue pour être accouplée coaxialement avec une canule (4) afin de réaliser une injection dans un vaisseau sanguin et possédant une tige (3b) ayant un axe longitudinal (A), une pointe distale (3a) et des moyensd'engagement transversaux (9) appliqué à proximité de ladite pointe (3a) ;
- un porte-canule (5), dans lequel ladite canule (4) s'étend depuis son extrémité distale, et qui définit une cavité interne (6) à son extrémité proximale opposée, pour permettre l'accouplement avec ledit porte-aiguille (2), et qui comporte des parois intérieures (7) ;
- un élément de protection (8) destiné à protéger ladite pointe d'aiguille (3a) et mobile entre une position de désactivation et une position d'activation, après l'emploi de la canule-aiguille (1) dans laquelle il est placé au-dessus de la pointe d'aiguille (3a) ;
ledit élément de protection (8), dans ladite position de désactivation, étant retenue de façon amovible par lesdites parois (7) de ladite cavité intérieure (6) dudit porte-canule (5), ledit élément de protection (8) comprenant un corps plat définissant :
- une paroi proximale transversale (10) à ladite aiguille, dans laquelle est formée une première ouverture de passage (14) de ladite tige (3b), qui a un périmètre de blocage desdits moyens d'engagement transversaux (9) ;
- un premier bras et un second bras (11, 12) s'étendant depuis ladite paroi proximale (10) vers ladite pointe distale (3a) ;
au moins ledit premier bras (11) définissant une seconde paroi (13) tournée transversalement vers ladite aiguille (3) et dans laquelle est formée une seconde ouverture de passage (15) de ladite tige (3b), ladite seconde ouverture de passage (15) étant alignée avec ladite première ouverture de passage (14) dans ladite position de désactivation et étant désalignée de ladite première ouverture de passage (14) dans ladite position d'activation, **caractérisée en ce que** ledit premier bras (11) définit deux cloisons latérales (16) repliées perpendiculairement à celui-ci et définit avec la seconde paroi (13) adjacente, une tête en boîtier (16a) possédant un côté entièrement ouvert faisant face à la paroi proximale (10).

2. Canule-aiguille selon la revendication 1, dans laquelle des cloisons de retenue amovibles (16) dudit élément de protection (8) sont interposés entre ladite cavité intérieure (6) et ledit élément de protection (8), et sont conçues pour le contraindre dans ladite position de désactivation et pour le relâcher vers ladite position d'activation.

3. Canule-aiguille selon la revendication 2, dans laquelle lesdits moyens de retenue comprennent une paire de cloisons latérales (16) s'étendant depuis au moins l'un desdits premier et second bras (11, 12) et convergeant vers ladite seconde paroi (13), lesdites cloisons latérales (16) possédant des bords de saisie sur lesdites parois intérieures (7) de ladite cavité intérieure (6).

4. Canule-aiguille selon la revendication 1, dans laquelle ledit premier bras (11), dans ladite position d'activation, est normalement oblique par rapport à ladite paroi proximale transversale (10).

5. Canule-aiguille selon la revendication 1, dans laquelle ledit second bras (12) est normalement perpendiculaire par rapport à ladite paroi proximale transversale (10).

6. Canule-aiguille selon n'importe laquelle des revendications précédentes, dans laquelle ledit élément de protection (8) est retiré de façon coulissante depuis ladite cavité intérieure (6) pendant le retrait de ladite aiguille (16), lorsque lesdits moyens d'engagement transversaux (9) sont bloqués contre ledit périmètre de blocage de ladite première ouverture de passage (14).
